(19) **Europäisches Patentamt / European Patent Office / Office européen des brevets**

(11) **EP 3 600 253 B1**

(12) # EUROPEAN PATENT SPECIFICATION

(45) Date of publication and mention
of the grant of the patent:
**16.03.2022 Bulletin 2022/11**

(21) Application number: **18714527.1**

(22) Date of filing: **03.04.2018**

(51) International Patent Classification (IPC):
**A61K 9/127** (2006.01)    **A61K 9/28** (2006.01)
**A61K 9/48** (2006.01)    **C07K 7/64** (2006.01)

(52) Cooperative Patent Classification (CPC):
**A61K 9/1271; A61K 9/2846; A61K 9/4891;**
**C07K 7/64**

(86) International application number:
**PCT/EP2018/058464**

(87) International publication number:
**WO 2018/178395 (04.10.2018 Gazette 2018/40)**

(54) **LIPOSOMAL COMPOSITIONS AND SOLID ORAL DOSAGE FORMS COMPRISING THE SAME**

LIPOSOMALE ZUSAMMENSETZUNGEN UND FESTSTOFFDOSIERUNGSFORMEN DAMIT

COMPOSITIONS LIPOSOMALES ET FORMES POSOLOGIQUES SOLIDES LES COMPRENANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.03.2017 EP 17000536**

(43) Date of publication of application:
**05.02.2020 Bulletin 2020/06**

(73) Proprietor: **Universität Heidelberg**
**69117 Heidelberg (DE)**

(72) Inventors:
• **UHL, Philipp**
**69124 Heidelberg (DE)**
• **SAUTER, Max**
**69115 Heidelberg (DE)**
• **MIER, Walter**
**64625 Bensheim (DE)**

(74) Representative: **Fuchs Patentanwälte**
**Partnerschaft mbB**
**Westhafenplatz 1**
**60327 Frankfurt am Main (DE)**

(56) References cited:
**CN-A- 103 417 480    US-A1- 2016 106 864**

• **SILVIA F. PANTZE ET AL: "Matrix liposomes: A solid liposomal formulation for oral administration : Solid liposomes for oral administration", EUROPEAN JOURNAL OF LIPID SCIENCE AND TECHNOLOGY., vol. 116, no. 9, September 2014 (2014-09), pages 1145-1154, XP055407942, DE ISSN: 1438-7697, DOI: 10.1002/ejlt.201300409**

• **ZHANG DONGDONG ET AL: "Cell-penetrating peptides as noninvasive transmembrane vectors for the development of novel multifunctional drug-delivery systems", JOURNAL OF CONTROLLED RELEASE, vol. 229, 16 March 2016 (2016-03-16), pages 130-139, XP029521834, ISSN: 0168-3659, DOI: 10.1016/J.JCONREL.2016.03.020**

• **BASHYAL SANTOSH ET AL: "Cell penetrating peptides as an innovative approach for drug delivery; then, present and the future", JOURNAL OF KOREAN PHARMACEUTICAL SCIENCES, KOREAN SOCIETY OF PHARMACEUTICAL SCIENCES AND TECHNOLOGY, KOREA, vol. 46, no. 3, 7 May 2016 (2016-05-07), pages 205-220, XP035971922, ISSN: 2093-5552, DOI: 10.1007/S40005-016-0253-0 [retrieved on 2016-05-07]**

SEQUENCE LISTING

<110> Universität Heidelberg

<120> Liposomal compositions and solid oral dosage forms comprising the same

<130> UH0001P-WO

<160> 8

<170> PatentIn version 3.5

<210> 1
<211> 16
<212> PRT
<213> Drosophila melanogaster

<400> 1

        Arg Gln Ile Lys Ile Trp Phe Gln Asn Arg Arg Met Lys Trp Lys Lys
        1                5                  10                  15

<210> 2
<211> 16
<212> PRT
<213> Human immunodeficiency virus type 1

<400> 2

        Cys Gly Arg Lys Lys Lys Arg Arg Gln Arg Arg Arg Pro Pro Gln Cys
        1                5                  10                  15

<210> 3
<211> 27
<212> PRT
<213> Artificial sequence

<220>
<223> artificially designed cell penetrating peptide

<400> 3

```
Gly Ala Leu Phe Leu Gly Phe Leu Gly Ala Ala Gly Ser Thr Met Gly
1               5                   10                  15


Ala Trp Ser Gln Pro Lys Ser Lys Arg Lys Val
            20                  25
```

<210> 4
<211> 9
<212> PRT
<213> Artificial sequence

<220>
<223> artificially designed cell penetrating peptide

<400> 4

```
Arg Arg Arg Arg Arg Arg Arg Arg Arg


        1                   5
```

<210> 5
<211> 18
<212> PRT
<213> Mus musculus

<220>
<221> MISC_FEATURE
<222> (18)..(18)
<223> K-amide

<400> 5

```
Leu Leu Ile Ile Leu Arg Arg Arg Ile Arg Lys Gln Ala His Ala His
1               5                   10                  15


Ser Lys
```

<210> 6
<211> 28
<212> PRT
<213> Homo sapiens

<220>
<221> MISC_FEATURE
<222> (28)..(28)
<223> L-amide

<400> 6

```
Gly Trp Thr Leu Asn Ser Ala Gly Tyr Leu Leu Gly Lys Ile Asn Leu
1               5                   10                  15
```

```
Lys Ala Leu Ala Ala Leu Ala Lys Ile Ser Ile Leu
            20                  25
```

<210> 7
<211> 27
<212> PRT
<213> Human immunodeficiency virus

<400> 7

```
Gly Ala Leu Phe Leu Gly Phe Leu Gly Ala Ala Gly Ser Thr Met Gly
1               5                   10                  15
```

```
Ala Trp Ser Gln Pro Lys Ser Lys Arg Lys Val
            20                  25
```

<210> 8
<211> 10
<212> PRT
<213> Artificial sequence

<220>
<223> artificially designed cell penetrating peptide

<400> 8

```
Arg Arg Arg Arg Arg Arg Arg Arg Arg Cys
1               5                   10
```

<210> 9
<211> 10
<212> PRT
<213> Artificial sequence

<220>
<223> artificially designed cell penetrating peptide

<400> 9

```
Arg Arg Arg Arg Arg Arg Arg Arg Arg Lys
1               5                   10
```

**Description**

The present invention relates to solid oral dosage forms comprising liposomes, said liposomes comprising conjugates of cell penetrating peptides (CPPs) and a compound, selected from a lipid and a fatty acid, wherein said liposomes are comprised in an enteric-coated capsule or enteric-coated tablet. The present invention further relates to said solid oral dosage forms for use in the oral delivery of therapeutic and diagnostic agents.

Oral drug delivery is considered as the most advantageous way of application, in particular for the treatment of chronic diseases, which demand long-term and repeated drug administration. The oral route offers high drug safety and is widely accepted among patients due to its convenience. Additionally, as sterility is not required for oral drug forms costs in production, storage and distribution is reduced, which may contribute to health care improvement in third world countries. It is estimated, that 90% of all marketed drug formulations are for oral use.

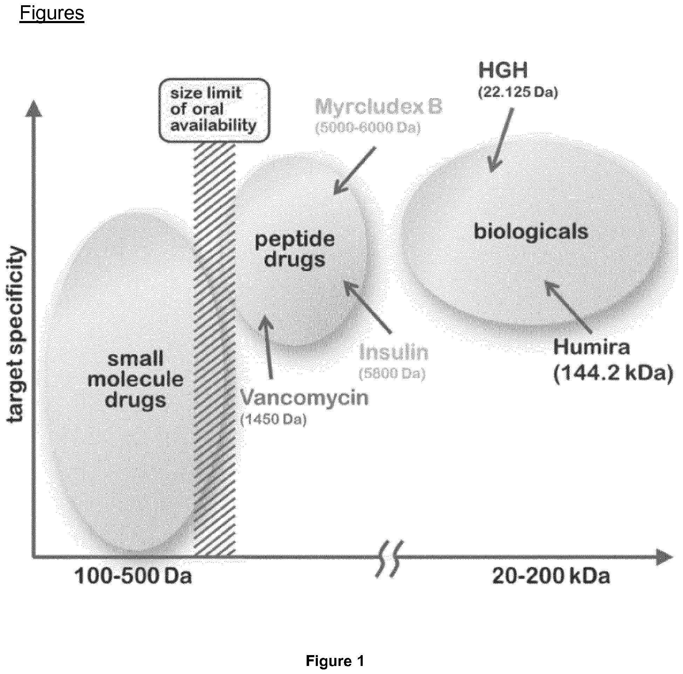

The number of macromolecular drugs, e.g. peptides, proteins, and antibodies, present in the pharmaceutical market is steadily increasing (Fig. 1). However, a limiting factor for these promising drugs is a poor oral bioavailability. Because of their sizes antibodies have particularly bad oral bioavailability. In particular, many macromolecular drugs show both a very poor stability under the acidic conditions in the stomach after oral administration and also poor absorption across the gastrointestinal barrier. Thus, such drugs have to be administered subcutaneously or intravenously which increases necessary medical efforts and causes increased costs, decreased patient compliance, and increased risk of complications.

To overcome this problem, different approaches to improve the bioavailability have been tested in the past years including solid lipid nanoparticles, nano- or micro-emulsions, and liposomes. However, conventional liposomal formulations have not been very convincing due to their instability in the gastrointestinal tract (GIT). Furthermore, some liposomal carrier systems comprising tetraether lipids have been devised which exhibit a significantly increased stability in the gastric environment. However, tetraether lipids can only be isolated from archaea and only on a laboratory scale, necessitating immense efforts and very high costs. Accordingly, tetraether lipids are available only in very limited amounts. Moreover, respective liposomal formulations are so far only available as suspensions showing a poor storage stability and patient compliance.

Pantze, Silvia F. et al: "Matrix liposomes: A solid liposomal formulation for oral administration : Solid liposomes for oral administration", Eur. J. Lipid Sci. Technol. 2014, 116, 1145-1154, discloses solid oral dosage forms comprising liposomes, suggesting the possibility of encapsulating the liposomes in enteric-coated capsules in order to provide stability in the gastrointestinal tract. Said dosage forms provide good oral bioavailability of macromolecular drugs such as peptides and proteins.

Accordingly, a technical problem underlying the present invention is to provide solid dosage forms for oral administration providing improved oral bioavailability of macromolecular drugs. The solution to the above technical problem is achieved by the embodiments characterized in the claims and set out in this description.

In particular, in a first aspect, the present invention relates to a solid oral dosage form comprising liposomes, said liposomes comprising a conjugate of

(a) at least one type of cell penetrating peptide (CPP), and
(b) a compound, selected from a lipid and a fatty acid;

wherein said conjugate is part of the liposome's lipid double layer;
wherein the dosage form is a gastro-resistant solid oral dosage form. Preferably, said liposomes are comprised in an enteric-coated capsule or enteric-coated tablet.

As used herein, the term "solid oral dosage form" relates to a solid dosage form for oral administration, *i.e.,* a capsule, a tablet, granules or pellets.

"Gastro-resistant" means that the dosage form does not substantially release its active agent - such as a therapeutic or diagnostic agent - in the acidic environment of a subject's stomach. Enteric coated formulations are gastro-resistant.

Further, the term "liposome" as used herein refers to artificially prepared vesicles composed of lipid bilayers. Liposomes can be used for delivery of agents due to their unique property of encapsulating a region of aqueous solution inside a lipophilic membrane. Lipophilic compounds can be dissolved in the lipid bilayer, and in this way liposomes can carry both lipophilic and hydrophilic compounds. To deliver the molecules to sites of action, the lipid bilayer can fuse with other bilayers such as cell membranes, thus delivering the liposome contents. By making liposomes in a solution of an agent, said agent can be delivered to the inner lumen of the liposome.

The liposomes used in the compositions according to the present invention are not particularly limited to specific lipids. In particular, the lipids used for the generation of said liposomes can be any suitable lipids known in the art. These lipids include - but are not restricted to - cholesterol or derivatives thereof, phospholipids, lysophospholipids, or combinations thereof. Accordingly, in a preferred embodiment, said liposomes comprise one or more lipids, selected from the group consisting of cholesterol and derivatives thereof, phospholipids, lysophospholipids, and combinations thereof. The cholesterol derivatives in the context of the present invention are steroids and compounds having a basic steroid molecular structure. Preferably, said liposomes comprise phospholipids, wherein said phospholipids can be synthetic, semi-synthetic or natural phospholipids, or combinations thereof. In general, suitable lipids can be selected from the group consisting of phosphatidylcholines, phosphatidylethanolamines, phosphati-dylinosites, phosphatidylserines, cephalines, phosphatidylglycerols, lysophospholipids, and combinations thereof. In a particular embodiment of the present invention, the liposomes comprise egg phosphatidylcholine (E-PC; lecithin) and cholesterol, preferably in an amount of about 85 to 95 mol-% E-PC, more preferably about 89 mol-% E-PC, and about 5 to 10 mol-% cholesterol. In an embodiment, the liposomes comprise egg phosphatidylcholine (E-PC; lecithin) and cholesterol in an amount of about 85 to 95 mol-% E-PC, more preferably about 89 mol-% E-PC, and about 5 to 10 mol-% cholesterol, wherein the relative amounts relate to the respective contents in the liposomes' bilayer. The liposomes to be used according to the present invention, as well as the solid oral dosage forms as such, may further comprise any further suitable agents known in the art such as e.g. enzyme inhibitors, permeation enhancers or other lipophilic or hydrophilic substances, or combinations thereof that can be used for the stabilization of liposomes or for altering liposome properties. Optional stabilizing agents comprise tetraether lipids. Such lipophilic or hydrophilic substances are not particularly limited and are known in the art. They include for example vitamin E, fatty acids, waxes, and mono-, di- and triglycerides, and mixtures thereof. Furthermore, substances that enhance the bioavailability of enclosed active agents, like enzyme inhibitors, tight junction modulators, chelating agents, or mixtures thereof can be added.

The conjugates comprised in the liposomes according to the present invention are conjugates of at least one type of cell penetrating peptide (CPP), and a compound, selected from a lipid and a fatty acid. The compound to which the CPPs are conjugated is preferably a suitable lipid as defined above, e.g. a lipid selected from the group consisting of cholesterol and derivatives thereof, phospholipids, lysophospholipids, and combinations thereof, wherein phospholipids are preferred, and wherein said lipids can be modified and/or activated lipids. Exemplary compounds in this respect are 1,2-dipal-mitoyl-sn-glycero-3-phosphoethanolamine-N-[4-(p-maleimidomethyl)cyclohexane-carboxamide] (sodium salt) or 1,2-dioleoyl-sn-glycero-3-phosphoethanol-amine-N-[4-(p-maleimidomethyl)cyclohexane-carboxamide] (sodium salt). Preferably, the CPPs are conjugated to said compound via a linker, such as a linker selected from the group consisting of bifunctional PEG-linkers known in the art. CPPs may be monomeric or dimerized. In this context, monomeric CPPs can be covalently conjugated to a phospholipid via a linker, or dimerized CPPs, wherein homo- and heterodimers are possible, are covalently conjugated to a phospholipid via a linker. Dimerization of CPPs can be effected by any means known in the art. In a particular embodiment, CPPs are dimerized via the tripeptide KAK. However, attachment is also possible without a linker, e.g. when using modified and/or activated lipids, such as e.g. phospholipids with a maleimide-modified headgroup.

Suitable phospholipids for the covalent conjugation of CPPs are not particularly limited to specific phospholipids. In particular, the phospholipids used for the covalent conjugation of CPPs can be any suitable phospholipids known in the art, wherein said phospholipids can be synthetic, semi-synthetic or natural phospholipids, or combinations thereof. In general, suitable phospholipids can be selected from the group consisting of phosphatidylcholines, phosphatidylethanolamines, phosphatidylinosites, phosphatidylserines, cephalines, phosphatidylglycerols, lysophospholipids, and combinations thereof. A particular phospholipid in this respect is egg phosphatidylcholine (E-PC; lecithin). Further, suitable phospholipids include PEG-modified versions of the above phospholipids, e.g. DSPE-PEG(2000) Maleimide (1,2-distearoyl-sn-glycero-3-phosphoethanolamine-N-[maleimide(polyethylene glycol)-2000] (ammonium salt)).

Suitable linkers for the covalent conjugation of CPPs to phospholipids are not particularly limited and are known in the art. They include for example bifunctional PEG-linkers in general; e.g. SM(PEG)$_{24}$ (PEGylated, long-chain SMCC crosslinker). Suitable exemplary linkers are SMCC (succinimidyl-4-(N-maleimidomethyl)cyclohexane-1-carboxylate)-linker, and 6-maleimido hexanoic acid linker. The length of the PEG moiety in such linkers influences encapsulation efficiency of drugs that may be incorporated into the liposomes comprised in the solid oral dosage forms of the present invention. Accordingly, said PEG moiety preferably has a length of 8 to 50 individual PEG units. Further, methods for covalently conjugating CPPs to phospholipids via linkers are not particularly limited and are known in the art.

In preferred embodiments, the liposomes present in the solid oral dosage forms of the present invention do not contain any tetraether lipids (TELs), which are specific lipids derived from archaea, e.g. the extremophilic archaeon *Sulfolobus acidocaldarius.*

According to the present invention, the solid oral dosage forms may further comprise at least one pharmaceutically acceptable excipient, and/or at least one protease inhibitor, and/or at least one lipase inhibitor within the solid oral dosage form such as the enteric-coated capsule or enteric-coated tablet. These can be present in the inner lumen of the liposomes, in the liposomes' lipid double layer (e.g. forming a part of the double layer by covalent or non-covalent attachment), or outside of the liposomes (e.g. in other parts of the dosage form). Preferably, said at least one pharmaceutically acceptable excipient is selected from the group consisting of sorbitan monostearate, tripalmitin, cetyl palmitate, alginate, ethyl oleate, C8 triglycerides, C10 triglycerides, cellulose, disaccharides, monosaccharides, oligosaccharides, magnesium stearate, corn starch, citric acid, tartaric acid, acid salts of amino acids, and combinations thereof. Furthermore, said at least one protease inhibitor is preferably selected from the group consisting of aprotinin, soybean trypsin inhibitor, bacitracin, sodium glycocholate, bestatin, leupeptin, cystatin, camostat mesilate, and combinations thereof. Furthermore, said at least one lipase inhibitor is preferably selected from the group, consisting of orlistat, lipstatin, chitin, chitosan, saponin, flavonoid glycoside, polyphenole, ebelacton A and B, esterastin, valilactone, panclicine, proanthocyanidin, vibralactone, and combinations thereof.

CPPs that can be used in connection with the present invention are not particularly limited and are known in the art. Preferably, the CPPs are CPPs showing a positive total charge. Further, CPPs according to the present invention can be linear or cyclized CPPs, wherein cyclized CPPs are particularly preferred. The terms "cyclized peptide", "cyclic peptide" and "cyclopeptide" are used synonymously herein.

More preferably, the CPPs are selected from the group consisting of

- penetratin (such as SEQ ID NO: 1; RQIKIWFQNRRMKWKK), derived from *Drosophila melanogaster,*
- TAT (transactivator of transcription)-peptide (such as SEQ ID NO: 2; CGRKKKRRQRRRPPQC), derived from HIV-1,
- MAP (model amphiphatic peptide) (such as SEQ ID NO: 3; GALFLGFLGAAGSTMGAWSQPKSKRKV), which is an artificial peptide,
- R9 (such as SEQ ID NO: 4; RRRRRRRRR), which is an artificial peptide,
- pVEC (such as SEQ ID NO: 5; LLIILRRRIRKQAHAHSK-amide), which is a CPP derived from murine vascular endothelial cadherin,
- transportan (such as SEQ ID NO: 6; GWTLNSAGYLLGKINLKALAALAKISIL-amide), which is derived from the human neuropeptide galanin, and
- MPG (such as SEQ ID NO: 7; GALFLGFLGAAGSTMGAWSQPKSKRKV), which is derived from HIV,

combinations thereof, and dimers thereof. In an embodiment the CPP is cyclized R9.

In this context, all of the above peptides can be present in a linear or in a cyclized form, wherein the cyclized form is preferred. Further, the CPPs can be composed of L-amino acids, D-amino acids, or mixtures thereof, wherein for linear CPPs, D-amino acids are preferred.

In contrast to linear CPPs, cell penetrating cyclopeptides are less susceptible to hydrolysis by peptidases, *i.e.,* they have been shown to be enzymatically more stable.

As used herein, the term "cyclized peptide" is not to be construed as relating to a peptide having one ring system only, *i.e.,* the present invention is not limited to monocyclic peptides. Accordingly, the present invention also relates to cyclopeptides wherein two or more ring systems are covalently linked to each other. Furthermore, the cyclopeptides may also comprise amino acids which are not part of the ring system. Thus, peptide side chains may be present in the cyclopeptides. Preferably, the cyclopeptides are monocyclic peptides, and more preferably monocyclic peptides having no peptide side chains.

In an embodiment, the cyclopeptides are positively charged. Thereby, mucosal uptake of the respective liposomes is enhanced. For example, the cyclopeptides as defined above may comprise mostly lysine and/or arginine moieties, which have isoelectric points of around 9.5 and 11, respectively. Due to their additional amino group, these two amino acids are positively charged under neutral and even under weakly basic conditions. Accordingly, a cyclopeptide mostly comprising moieties of said two specific amino acids is positively charged under neutral and weakly basic conditions as well. Herein, the term "mostly comprising" means that at least 50%, preferably at least 60%, more preferably at least 70%, and particularly preferably at least 80% of the amino acids forming a cyclopeptide molecule are lysine and/or arginine moieties. Thereby, it is ensured that the cyclopeptides have a positive charge under neutral and weakly basic conditions, *i.e.,* have an isoelectric point of more than 7. Therefore, in a specific embodiment of the present invention, the cyclopeptides of the above-defined liposomes comprised in the solid oral dosage forms of the present invention have an isoelectric point of more than 7.0, preferably of more than 7.5, more preferably of more than 8.0, and particularly preferably of more than 8.5. In this context, the isoelectric point of the cyclopeptide is the arithmetic mean of the isoelectric points of the amino acids forming the cyclopeptide.

In a specific embodiment of the present invention, the cyclopeptides comprise between 2 to 19, preferably between 3 to 16, more preferably between 4 to 14, and particularly preferably between 6 to 12 arginine moieties as well as one moiety selected from the group consisting of tyrosine, threonine, serine and cysteine. For example, the cyclopeptides may comprise nine arginine moieties and one cysteine moiety in the ring system, and are referred to as a cyclic cysteine R9 derivative (such as SEQ ID NO: 8; RRRRRRRRRC). Another preferred example is a cyclopeptide comprising nine arginine moieties and one lysine moiety in the ring system, which is referred to as R9K derivative (SEQ ID NO: 9; RRRRRRRRRK).

The amino acids forming the cyclopeptides are not limited to proteinogenic amino acids. Herein, the amino acids may be selected from any amino acids known in the art, and may include the respective D-enantiomer, L-enantiomer, or any mixture thereof. Herein, the amino acids may be further functionalized so as to covalently attach to the modified biodegradable polymer chains.

According to the present invention, the CPP-conjugates are part of the liposome's lipid double layer. In this context, the term "being part of the liposome's lipid double layer" is intended to indicate the fact that said conjugates are integrated into said lipid double layer via their lipid and/or fatty acid portion, *i.e.,* said lipid and/or fatty acid portion is contained in the lipid double layer, whereas the CPP portion of the conjugate is presented at the surface of the liposome.

Preferably, the liposomes comprised in the solid oral dosage forms of the present invention comprise said CPPs in an amount of 0.05 to 5 mol-%, or 0.05 to 3 mol-%, preferably 0.05 to 2 mol-%, and more preferably 0.1 to 1 mol-%, based on the total lipid and/or fatty acid amount. In the case of monomeric CPPs, these are preferably comprised in an amount of 0.05 to 2 mol-%, preferably 0.1 to 1 mol-%, based on the total lipid and/or fatty acid amount. In the case of dimerized CPPs, these are preferably comprised in an amount of 0.05 to 0.5 mol-%, preferably 0.1 mol-%, based on the total lipid and/or fatty acid amount. It has been found that the polydispersity index of the liposomes increases with increasing amount of CPP in case of high proportions of CPP. If the amount of CPP is too high, no liposomes will be formed due to the high positive charge of the CPPs which will lead to high repulsion forces.

The lipids and/or fatty acids used for preparation of the liposomes can also be attached to target seeking structures such as peptide sequences, antibodies, receptor ligands, surfactants and/or combinations thereof.

In a preferred embodiment of the present invention, the liposomes comprised in the solid oral dosage forms of the present invention are lyophilized. Means for the lyophilization of liposomes are not particularly limited and are known in the art. Advantageously, the liposomes can be freeze-dried, e.g. using 300 to 500 mM sucrose as a lyoprotector. Lyophilization as defined above enables the long-term storage of the solid oral dosage forms of the present invention.

In a preferred embodiment, the liposomes comprised in the solid oral dosage forms of the present invention exhibit a Z-Average measured by dynamic light scattering after dilution in aqueous medium of at most 350 nm and a polydispersity index (PDI) of at most 0.3, where a Z-Average of 100 to 200 nm and a polydispersity index of 0.1 to 0.3, preferably about 0.2 is particularly preferred.

Methods for the generation of liposomes are not particularly limited and are known in the art. They include for example high pressure homogenization, extrusion, ethanol injection and dual asymmetric centrifugation (DAC).

In preferred embodiments, the solid oral dosage forms of the present invention can further comprise at least one therapeutic agent and/or at least one diagnostic agent within the dosage form, such as in the enteric-coated capsule or enteric-coated tablet.

Respective therapeutic agents are not particularly limited and include any agents for which oral delivery might be of interest. They include for example macromolecules such as peptidic drugs (e.g. vancomycin, glatiramer acetate, Myrcludex B, octreotide, all sorts of insulin, and liraglutide, as well as other GLP (glucagon-like peptide)-analogues such as exenatide, lixisenatide, albiglutide, dulaglutide, taspoglutide, and semaglutide), and proteins or antibodies (e.g. etanercept; pegfilgrastim; adalimumab, infliximab, rituximab, epoietin alfa, tratuzumab, ranibizumab, beta-interferon, omalizumab). Other examples include pharmaceutically active agents selected from the group consisting of human growth hormone, growth hormone releasing hormone, growth hormone releasing peptide, interferons, colony stimulating factors, interleukins, macrophage activating factor, macrophage peptide, B cell factor, T cell factor, protein A, allergy inhibitor, cell necrosis glycoproteins, immunotoxin, lymphotoxin, tumor necrosis factor, tumor suppressors, metastasis growth factor, alpha-1 antitrypsin, albumin and fragment polypeptides thereof, apolipoprotein-E, erythropoietin, factor VII, factor VIII, factor IX, plasminogen activating factor, urokinase, streptokinase, protein C, C-reactive protein, renin inhibitor, collagenase inhibitor, superoxide dismutase, platelet-derived growth factor, epidermal growth factor, osteogenic growth factor, bone stimulating protein, calcitonin, insulin, atriopeptin, cartilage inducing factor, connective tissue activating factor, follicle stimulating hormone, luteinizing hormone, luteinizing hormone releasing hormone, nerve growth factors, parathyroid hormone, relaxin, secretin, somato-medin, insulin-like growth factor, adrenocortical hormone, glucagon, cholecystokinin, pancreatic polypeptide, gastrin releasing peptide, corticotropin releasing factor, thyroid stimulating hormone, monoclonal or polyclonal antibodies against various viruses, bacteria, or toxins, virus-derived vaccine antigens, octreotide, cyclosporine, rifampycin, lopinavir, ritonavir, vancomycin, telavancin, oritavancin, dalbavancin, bisphosphonates, itraconazole, danazol, paclitaxel, cyclosporin, naproxen, capsaicin, albuterol sulfate, terbutaline sulfate, diphenhydramine hydrochloride, chlorpheniramine maleate, loratidine hydrochloride, fexofenadine hydrochloride, phenylbutazone, nifedipine, carbamazepine, naproxen, cyclosporin, betamethasone, danazol, dexamethasone, prednisone, hydrocortisone, 17 beta-estradiol, ketoconazole, mefenamic acid, beclomethasone, alprazolam, midazolam, miconazole, ibuprofen, ketoprofen, prednisolone, methylprednisone, phenytoin, testosterone, flunisolide, diflunisal, budesonide, fluticasone, insulin, glucagon-like peptide, C-Peptide, erythropoietin, calcitonin, lutenizing hormone, prolactin, adrenocorticotropic hormone, leuprolide, interferon alpha-2b, interferon beta-la, sargramostim, aldesleukin, interferon alpha-2a, interferon alpha-n3alpha-proteinase inhibitor, etidronate, nafarelin, chorionic gonadotropin, prostaglandin E2, epoprostenol, acarbose, metformin, desmopressin, cyclodextrin, antibiotics, antifungal drugs, steroids, anticancer drugs, analgesics, anti-inflammatory agents, anthelmintics, anti-arrhythmic agents, penicillins, anticoagulants, antidepressants, antidiabetic agents, antiepileptics, antihistamines, antihypertensive agents, antimuscarinic agents, antimycobacterial agents, antineoplastic agents, CNS-active agents, immunosuppressants, antithyroid agents, antiviral agents, anxiolytic sedatives, hypnotics, neuroleptics, astringents, beta-adrenoceptor blocking agents, blood products and substitutes, cardiacinotropic agents, contrast media, corticosteroids, cough suppressants, expectorants, mucolytics, diuretics, dopaminergics, antiparkinsonian agents, haemostatics, immunological agents, lipid regulating agents, muscle relaxants, parasympathomimetics, parathyroid calcitonin, prostaglandins, radiopharmaceuticals, sex hormones, steroids, anti-allergic agents, stimulants, anoretics, sympathomimetics, thyroid agents, vasidilators, xanthines, heparins, therapeutic oligonucleotides, somatostatins and analogues thereof, and pharmacologically acceptable organic and inorganic salts or metal complexes thereof.

Further, respective diagnostic agents are not particularly limited and include any agents for which oral delivery might be interesting.

The above agents may be present in the solid oral dosage forms of the present invention enclosed in the liposomes, *i.e.,* in the inner lumen of said liposomes, e.g. when said agents are hydrophilic, or integrated into the liposomal membrane, e.g. when said agents are lipophilic. In this context, the encapsulation of therapeutic and/or diagnostic agents depends on the hydrophilicity of said agents and the liposome preparation method.

In a preferred embodiment, the content of therapeutic and/or diagnostic agent in the solid oral dosage forms according to the present invention is above 0 and at most 50 % (w/w) with respect to the solid oral dosage form, preferably between 5 and 20% (w/w). Enteric coatings, *i.e.,* coatings that are resistant to gastric juice, for use in the context of the present invention are not particularly limited and are known in the art. An exemplary coating contains 3% (w/w) water, 1.25% (w/w) triacetin, 82.75% (w/w) isopropanol, and 13% (w/w) Eudragit L 100. In this context, Eudragit L 100 is an anionic copolymer of methacrylic acid and ethyl acrylate in an acid to ester ratio of 1:1.

The solid oral dosage forms of the present invention can be for use in medicine. The dosage forms of this invention are particularly useful for the treatment of chronic diseases that require regular administration of therapeutic agents. Preferably, said solid oral dosage forms are for use in the treatment of sepsis, diabetes, rheumatism, acromegaly, all kinds of hepatitis, all kinds of cancer, or anemia. Further, the dosage forms may be used for treatment of autoimmune diseases, diseases that require administration of immunosuppressants , infectious diseases, diseases that require administration of growth hormones, enzyme-deficiency diseases, or neurodegenerative diseases (i.e. Alzheimer's disease, Parkinson's disease).

In a related aspect, the present invention relates to the solid oral dosage form of the present invention for use in the oral delivery of at least one therapeutic agent and/or at least one diagnostic agent. The invention further includes the solid oral dosage form for use in a method of treating a subject in need thereof comprising

- administering to the subject an amount of the solid oral dosage form of this invention which is sufficient to achieve the desired therapeutic effect by oral delivery.

The subject may be a mammal, such as a human.

In this context, the term "oral delivery" relates to the delivery of one or more agents by way of oral administration of said dosage forms.

In this aspect, all relevant limitations and definitions provided for the other aspects of the present invention apply in an analogous manner. In particular, the solid oral dosage forms, therapeutic agents, and diagnostic agents are as defined above.

In a further related aspect, the present invention relates to the solid oral dosage form for use in a method of delivering at least one therapeutic agent and/or at least one diagnostic agent to a subject, comprising the step of administering, preferably orally administering, a solid oral dosage form of the present invention to said subject.

In this aspect, all relevant limitations and definitions provided for the other aspects of the present invention apply in an analogous manner. In particular, the solid oral dosage forms, therapeutic agents, and diagnostic agents are as defined above.

As used herein, the term "about" is intended to be a modifier of $\pm$ 10% of the specified value. As an example, the term "about 5%" is intended to encompass the range of 4.5 to 5.5%.

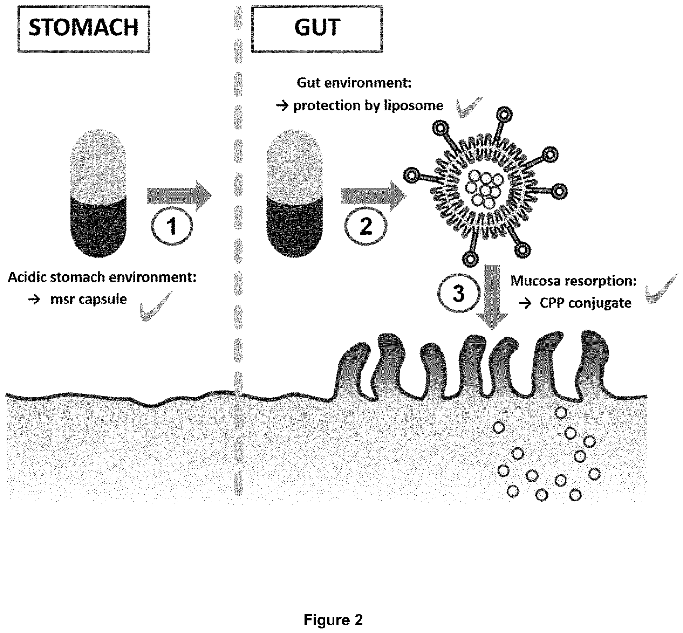

The present invention advantageously provides *inter alia* solid oral dosage forms comprising CPP-coupled liposomes for oral administration, said liposomes for example being comprised in an enteric-coated capsule or enteric-coated tablet. The CPPs significantly increase resorption of the liposomes at the intestinal mucosa, whereas the gastro-resistant dosage form ensures gastric passage (Fig. 2). Thus, a significant increase of the oral bioavailability of drugs comprised in said liposomes is achieved. This provides the possibility of oral administration for a wide range of macromolecular drugs which in turn increases patient compliance. Further, lyophilization of the liposomes increases storage stability of respective dosage forms.

Brief description of the figures

The figures show:

| | |
|---|---|
| **Figure 1:** | In the prior art, only drugs with a molecular weight of <500 Da could be administered orally. Most of the drugs with higher molecular weight, e.g. peptides and biologicals, must be administered parenterally. |
| **Figure 2:** | Liposomes coated with CPPs are administered orally. Therefore, the liposomes are lyophilized and transferred into a solid dosage form. Gastric passage is ensured by an enteric coating of the dosage form. Further, resorption of liposomes at the intestinal mucosa is significantly increased by the CPPs. |
| **Figure 3:** | Ussing chamber studies of liposomes coated with the cyclic R9 CPP in contrast to free vancomycin. |
| **Figure 4:** | Blood levels of vancomycin after oral application of free vancomycin and vancomycin incorporated into liposomes containing the cyclic R9 CPP in Wistar rats. |
| **Figure 5:** | Size and PDI of liposomes containing 0.1-1 mol-% of the CPP penetratin. |
| **Figure 6:** | Size and PDI of liposomes containing 0.1-1 mol-% of the CPP MAP. |
| **Figure 7:** | Zetapotentials of liposomes containing 0.1-1 mol-% of the CPP Penetratin. |
| **Figure 8:** | Zetapotentials of liposomes containing 0.1-1 mol-% of the CPP MAP. |
| **Figures 9:** | Synthesis of preferred, exemplary conjugates. |
| **Figures 10:** | Synthesis of preferred, exemplary conjugates. |
| **Figure 11:** | Structure of a modified phospholipid used to make a conjugate. |
| **Figure 12:** | Exemplary conjugate used in animal studies, and modified phospholipid. |
| **Figure 13:** | Further exemplary conjugate, and modified phospholipid. |
| **Figure 14:** | PDI and size of different liposomes. |
| **Figure 15:** | PDI and size of vancomycin-loaded liposomes after lyophilisation. |
| **Figure 16:** | PDI and size of liraglutide-loaded liposomes after lyophilisation. |
| **Figure 17:** | Encapsulation efficiencies for the vancomycin and liraglutide. |
| **Figure 18:** | Zetapotential of liposomal formulations prepared according to example 7. |
| **Figure 19:** | Comparison of AUC values for adalimumab intravenous and oral in dogs. |

Examples

The present invention will be further illustrated in the following examples without being limited thereto.

**1. Preparation of liposomes**

Liposomes were prepared by the film method with subsequent dual asymmetric centrifugation using a Speed-Mixer™. Chloroform/methanol 9:1 (v/v) was used as the solvent. The organic solvent was evaporated by a nitrogen stream. The resulting lipid film was dried for 1 h in a vacuum chamber. Afterward 20 mg of glass beads were added. The liposomes were prepared by speed mixing at 3000 rpm in a dual asymmetric centrifuge using a special vial holder. Three runs were performed and different amounts of PBS were added as shown in the following table.

| | Time [min] | Volume added |
|---|---|---|
| Run 1 | 20 | 50 µl PBS + API |
| Run 2 | 5 | 100 µl PBS + API |
| Run 3 | 5 | 100 µl PBS |

**2. Preparation of conjugates**

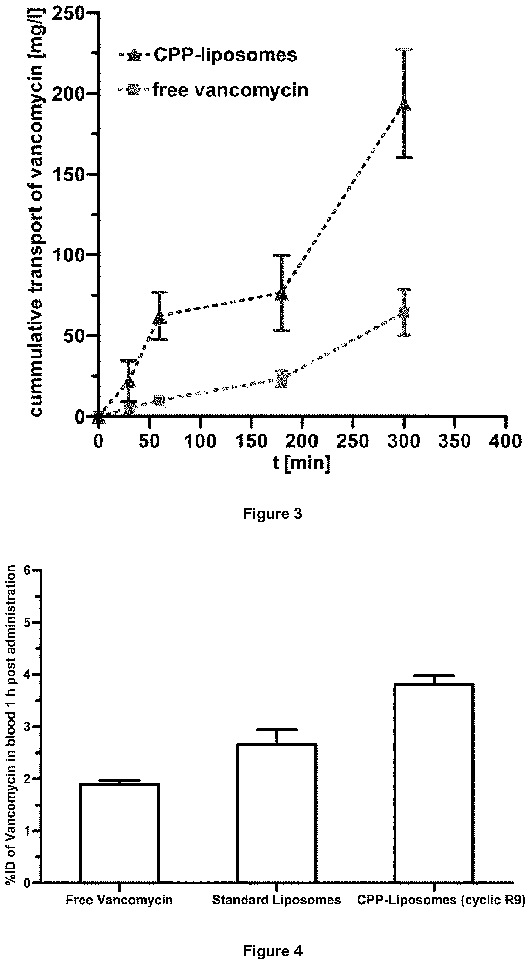

a. Conjugates of examples shown in Figures 3 and 4

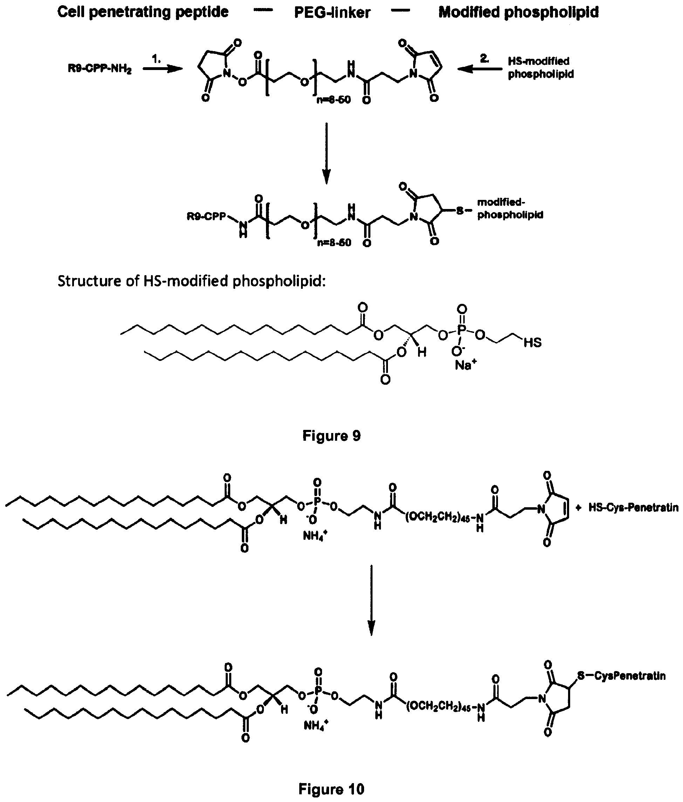

Figure 9 illustrates the synthesis of preferred, exemplary conjugates, in particular those used in the examples shown in Figures 3 and 4. The linker used in this example was SM(PEG)8, a PEGylated, long-chain SMCC crosslinker, and succinimidyl([N-maleimidopropionamido]-ethyleneglycol)ester, respectively. R9-CPP stands for either the linear or cyclic nona-arginine peptide. In case of the cyclic it is cyclized via a lysine (R9K) and coupled at the side chain amino function of this lysine.

For coupling of cyclic CPP to the bifunctional PEG-linker, as a first step the cyclized CPP was coupled by an additional lysine to the linker (1.). For this reaction an excess of the CPP was used. In the second step this intermediate product was coupled to the thiol modified phospholipid (2.).The modified phospholipid was 1,2-dipalmitoyl-sn-glycero-3-phosphothioethanol, sodium salt.

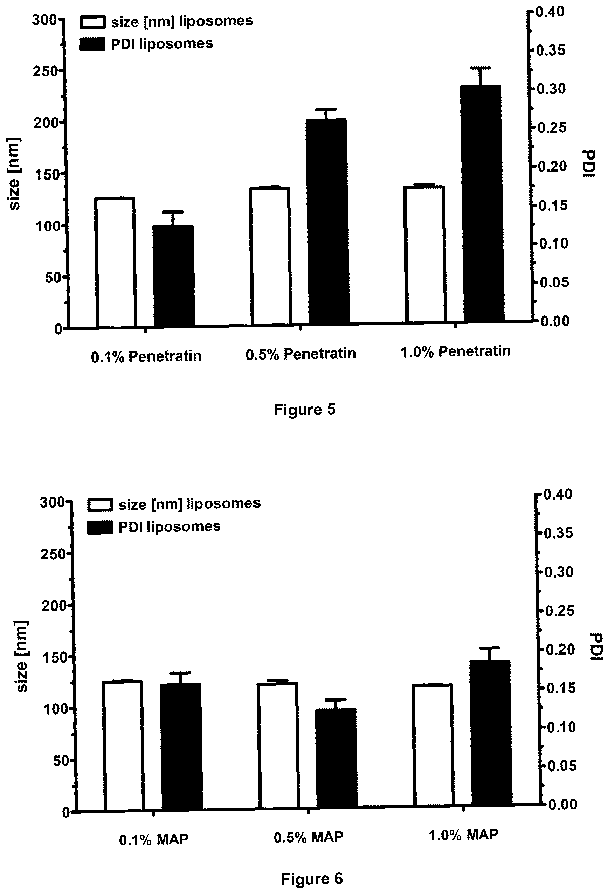

### b. Conjugates of examples shown in Figures 5-8

Figures 10 illustrates the synthesis of preferred, exemplary conjugates, in particular those used in the examples shown in Figures 5 to 8.

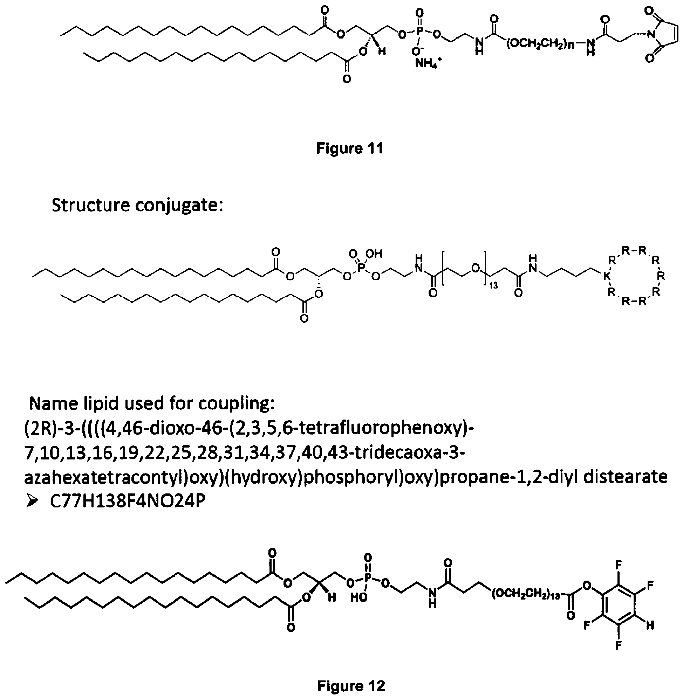

Cysteine-modified penetratin was coupled to the headgroup-modified phospholipid which is shown in Figure 11. Its chemical name is 1,2-distearoyl-sn-glycero-3-phosphoethanolamine-N-[maleimide(polyethylene glycol)-2000], ammonium salt.

### c. Conjugate used in animal studies

Figure 12 shows a conjugate that was used in liposomes for animal studies, and a modified phospholipid used to make the conjugate.

### d. A further conjugate

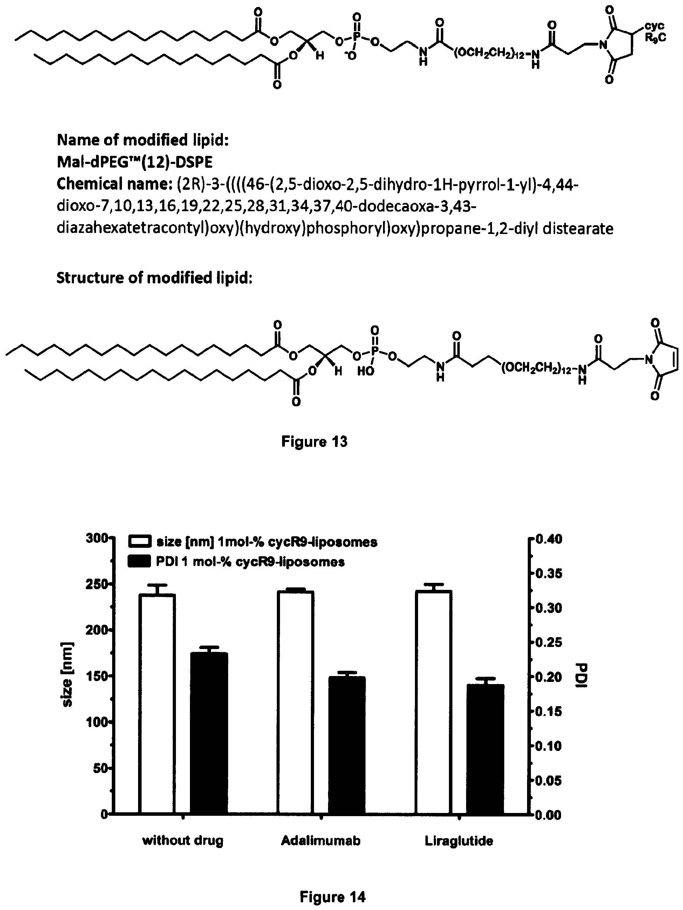

A further conjugate comprising cyclic R9C was prepared. The conjugate prepared and the modified lipid used to make it are shown in Figure 13.

## 3. Determination of blood levels

Blood levels of vancomycin were assessed in Wistar rats after oral administration of the drug. The animal trial compared the oral bioavailability of liposomes containing cell penetrating peptides (CPPs) conjugated as shown in Figure 9 to free vancomycin after oral application. Standard liposomes served as control and consisted of lecithin and cholesterol. Blood levels of vancomycin were determined by immunoassay. 6 h before oral application, the food of the rats was removed. For oral application, the rats were narcotized by use of isoflurane and oral application took place by gavage. 1 h after oral administration, the rats were sacrificed and the blood levels of vancomycin were determined. The results are shown in Figure 4.

The animal trial shows a significant increase in the oral availability of vancomycin by the use of CPP-liposomes in comparison with the free peptide and standard liposomes (Fig. 4).

## 4. Ussing chamber trial

For the Ussing chamber trial, a liposomal formulation containing 1 mol-% of the cyclic R9-derivative shown in Figure 9 was used and compared to the free peptide (vancomycin). The liposomes were prepared in a phosphate buffer pH = 6.8 as explained above in example 1. For this experiment, the small intestine of sacrificed rats (Sprague Dawley) was rinsed with NaCl and afterwards fixed in the Ussing chamber (0.64 $cm^2$ surface; Firma NaviCyte, MA1 66-00XX). Afterwards, the chambers were filled with incubation medium (Krebs-Ringer buffer) and equivalent amounts of vancomycin in the liposomal formulation and the free peptide were added. The trial was performed over 5 h. At determined time points, samples were taken of the acceptor chamber und replaced by Krebs-Ringer buffer. The cumulative amount of vancomycin in the samples was determined. The results are shown in Figure 3.

The Ussing chamber studies showed a strong increase in the transport of vancomycin over the mucosal barrier for the CPP-liposomes in contrast to the free peptide. Therefore it can be assumed that the CPP-liposomes strongly enhance the transport of substances through mucosal barriers (Fig. 3).

## 5. Particle size and PDI

The particle size and PDI of all liposomal formulations were determined at room temperature using a Zetasizer Nano ZS from Malvern™ (Malvern Instruments Ltd., Worcestershire, United Kingdom). Size and PDI were measured after dilution to a lipid concentration of 0.076 mg/ml with a 10 mM phosphate buffer with a pH of 7.4 using the automatic mode.

Figure 5 illustrates size and PDI of liposomes containing 0.1-1 mol-% of the CPP penetratin. The liposomal size remains nearly constant while the PDI shows an increase by the use of higher amounts of Penetratin.

Figure 6 shows size and PDI of liposomes containing 0.1-1 mol-% of the CPP MAP. The amount of the CPP incorporated into the liposomes did not influence liposomal size and PDI.

## 6. Zetapotential

The zetapotential of all liposomal formulations was determined at room temperature using a Zetasizer Nano ZS from Malvern™ (Malvern Instruments Ltd., Worcestershire, United Kingdom). The zetapotential was determined after dilution to a lipid concentration of 0.95 mg/ml by a 50 mM phosphate buffer with a pH of 7.4. The default settings of the automatic mode of the Zetasizer Nano ZS from Malvern™ (Malvern Instruments Ltd., Worcestershire, United Kingdom) were the following: number of measurements = 3; run duration = 10 s; number of runs = 10; equilibration time = 60 s; refractive index solvent 1.330; refractive index polystyrene cuvette 1.590; viscosity = 0.8872 mPa s; temperature = 25 °C; dielectric constant = 78.5 F/m; backscattering mode (173°); automatic voltage selection; Smoluchowski equation.

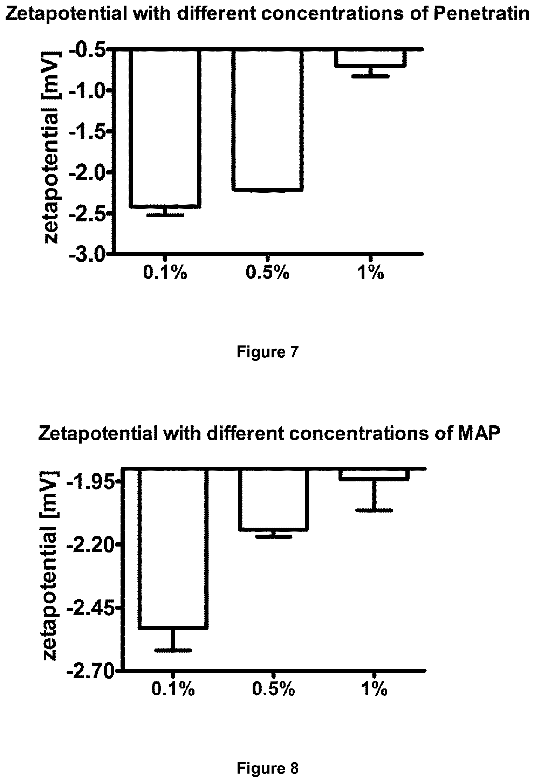

Figure 7 compares the zetapotentials of liposomes containing 0.1-1 mol-% of the CPP Penetratin. The higher the amount of the CPP, the higher was the zetapotential due to the positive charge of the CPP.

Figure 8 illustrates the zetapotentials of liposomes containing 0.1-1 mol-% of the CPP MAP. The higher the amount of the CPP, the higher was the zetapotential. Generally, a zetapotential in the range of from -5 to 10 mV is desirable, with -3 to 7 mV being preferred. If possible, the zetapotential should be positive. However, if the zetapotential is too high, the liposomes will not be stable.

### 7. Preparation of conjugate for animal studies (adalimumab in dogs)

Peptide synthesis of cyclized R9 was carried out on a Chloro-(2'-chloro)trityl Polystyrene resin with a capacity of 0,89 mmol/g. Loading was done with 0,8 mmol Fmoc-Lys(Boc)-OH per gram resin in DCM with 3 equivalents of DIPEA for 2 h. Excess 2-chlorotrityl-functions were quenched by MeOH-loading with a mixture of DCM/MeOH/DIPEA of 17:2:1. Nine consecutive steps of coupling Fmoc-Arg(Pbf)-OH followed in DMF, with an excess of 7 equivalents amino acid; 6.6 equivalents HBTU and 4 equivalents DIPEA. Fmoc-groups were removed by treatment with 20 % piperidine in DMF. In between steps the resin was washed rigorously with DMF.

Cleavage of the side chain protected peptide was achieved by a mixture of DCM/Trifluoroethanol/acetic acid of 7:2:1. The cleavage solution was co-evaporated with toluene three times on a rotary evaporator. The side chain protected peptide was dissolved in DMF in a concentration of 3 mg/ml. Cyclization was performed with 4 equivalents of PyAOP and DIPEA at RT overnight. After stopping the reaction with water the solution was concentrated to a fiftieth to hundredth of the starting volume and the side chain protected cyclo-peptide precipitated by pouring into cold tButyl-methylether.

The precipitated protected cyclo-peptide was dried and subsequently deprotected with a mixture of 5 % Dithioethanole in TFA. After precipitation with diethylether the cyclo-peptide was purified.

The resulting peptide was then used to make conjugates and liposomes as described before. The liposomes were loaded with adalimumab and liraglutide, respectively. Control liposomes without drug were prepared as well.

Size and PDI of the liposomes are shown in Figure 14.

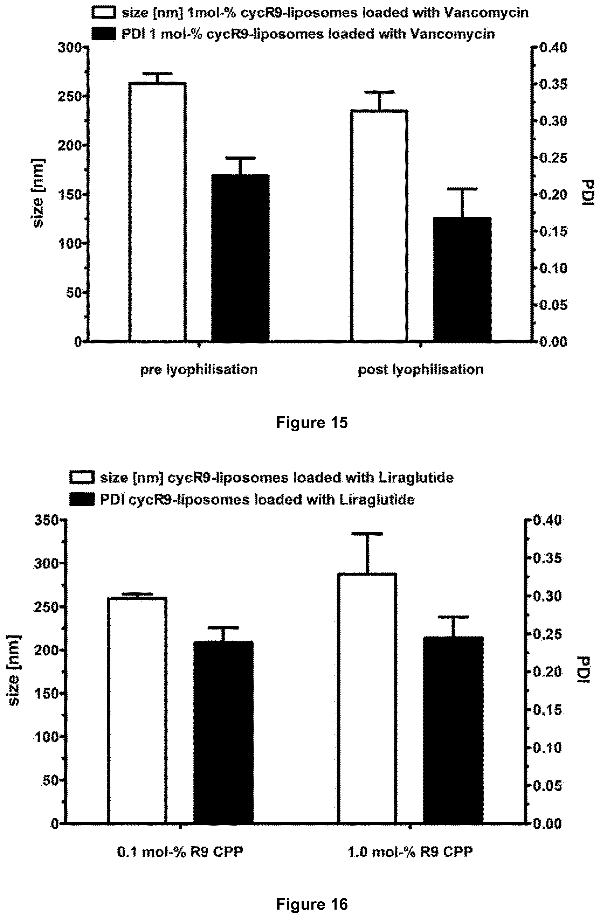

The liposomes were lyophilized. PDI and size of vancomycin-loaded liposomes after lyophilisation are shown in Figure 15. PDI and size of vancomycin-loaded liposomes after lyophilisation are shown in Figure 16. Lyophilisation did not have any significant effect on size or PDI.

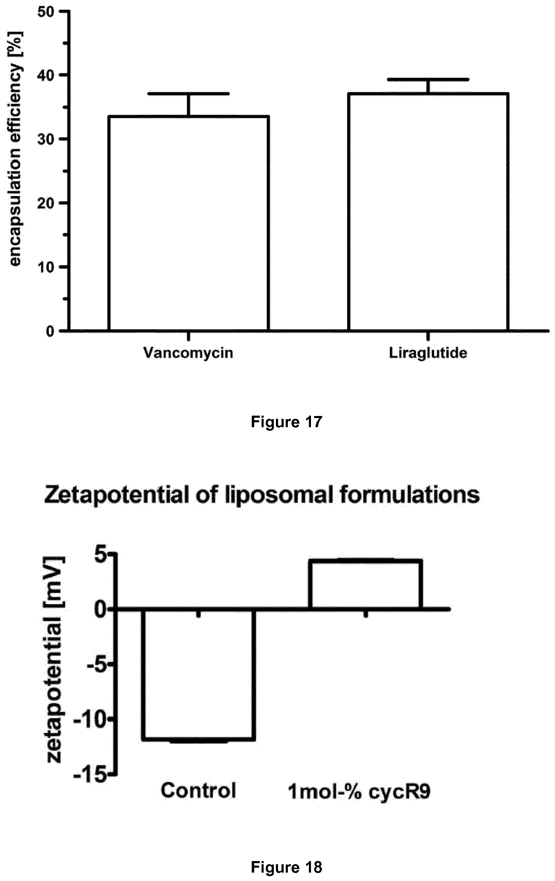

Zetapotential of these liposomal formulations are illustrated in Figure 18. Due to the high positive charge of the CPP-conjugate, the cycR9-CPP-liposomes show a positive zetapotential in contrast to the control liposomes.

### 8. Encapsulation efficiencies

The encapsulation efficiencies of all peptide model substances was determined by reversed phase HPLC (Agilent 1100 Series) using a C18 column (Chromolith® Performance RP-18e, 100-3 mm) applying a linear gradient of 0.1% TFA in water (eluent A) to 0.1% TFA in acetonitrile (eluent B) within 5 min (flow rate 2 ml/min; UV absorbance $\lambda$ = 214 nm). After the speed mixing process, the liposomes were divided into two parts with 100 $\mu$l each. Part 1 was used to calculate the 100% value obtained by destroying the liposomes by the addition of 50 $\mu$l 1% Triton™ X-100 and determining the area under the curve (AUC) of the model substance by HPLC. Part 2 was purified by Sephadex G-25 gel filtration chromatography (NAP™-5 columns) and quantified in the same way as part 1. The encapsulation efficiency E(%) was calculated using the following equation:

$$E(\%)=([AUC]\text{model substance part2}/[AUC]\text{ model substance part1}) \times 100\%$$

whereby [AUC] model substance part 2 is the concentration of model substance in the purified liposomal fraction and [AUC] model substance part 1 is the concentration of the model substance in the liposomal suspension

Figure 17 compares the results for both model substances.

**9. Animal studies**

Single dose PK study of oral Adalimumab (Humira™) in comparison with Adalimumab after intravenous administration. As animals, 2 beagle dogs were used. The oral dose of Adalimumab was 15 mg encapsulated in liposomes containing 1-mol% of the cycR9 conjugate described before. The liposomes were lyophilized and afterwards filled in an enteric coated capsule. The intravenous dose was 3 mg. The blood sampling time points for the oral administration were: 0 (pre-dose), 0:15, 0:30, 1:00, 2:00, 4:00, 8:00, 24:00, 48:00, 96:00 h p.a. The blood sampling time points for the intravenous administration were: 0 (pre-dose), 0:15, 0:30, 1:00, 2:00, 4:00, 8:00, 24:00, 48:00, 96:00 h p.a. All blood samples were analyzed by ELISA measurements (IDKmonitor® Adalimumab drug level ELISA, Immundiagnostik AG, Bensheim).

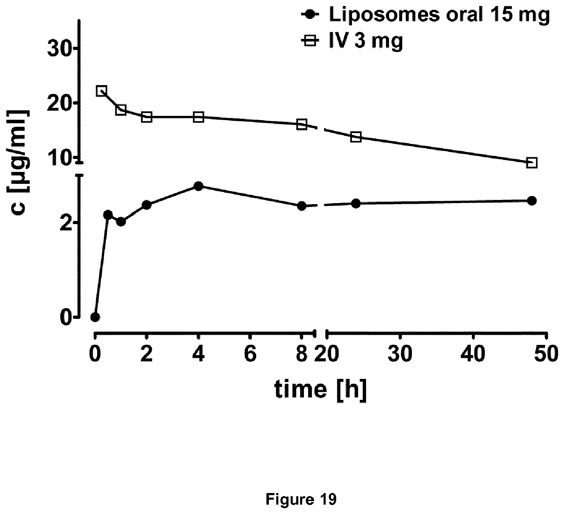

The results are illustrated in Figure 19. As results, by comparison of AUC values, a 3.55 % bioavailability of Adalimumab after oral administration could be obtained. These results demonstrate the enormous potential of this liposomal formulation for the oral delivery of macromolecular agents. To our knowledge, such a high bioavailability for oral administration of antibodies has not been reported before.

**Claims**

1. A solid oral dosage form comprising liposomes, said liposomes comprising a conjugate of

   (a) at least one type of cell penetrating peptide (CPP), and
   (b) a compound, selected from a lipid and a fatty acid;

   wherein said conjugate is part of the liposome's lipid double layer;
   wherein the dosage form is a gastro-resistant solid oral dosage form.

2.  The solid oral dosage form of claim 1, wherein said liposomes are comprised in an enteric-coated capsule or enteric-coated tablet.

3.  The solid oral dosage form according to claim 1 or 2, further comprising at least one pharmaceutically acceptable excipient, and/or at least one protease inhibitor, and/or at least one lipase inhibitor within the solid oral dosage form.

4.  The solid oral dosage form according to one or more of claims 1 to 3, wherein said at least one type of CPP is a cyclized CPP.

5.  The solid oral dosage form according to one or more of claims 1 to 4, wherein said at least one type of CPP is selected from the group consisting of linear or cyclized penetratin, linear or cyclized transactivator of transcription (TAT)-peptide, linear or cyclized model amphiphatic peptide (MAP), linear or cyclized R9, linear or cyclized pVEC, linear or cyclized transportan, linear or cyclized MPG, combinations thereof, and dimers thereof.

6.  The solid oral dosage form according to one or more of claims 1 to 5, wherein said liposomes comprise said at least one type of CPP in an amount of 0.05 to 5 mol-% based on the total lipid and/or fatty acid amount.

7.  The solid oral dosage form according to one or more of claims 1 to 6, wherein the compound to which said at least one type of CPP is conjugated is selected from the group consisting of cholesterol and derivatives thereof, phospholipids, lysophospholipids, and combinations thereof, wherein the cholesterol derivatives are steroids and compounds having a basic steroid molecular structure.

8.  The solid oral dosage form according to one or more of claims 1 to 7, wherein said at least one type of CPP is conjugated to said compound via a bifunctional PEG-linker.

9.  The solid oral dosage form according to one or more of claims 1 to 8, wherein said liposomes are lyophilized.

10. The solid oral dosage form according to one or more of claims 1 to 9, further comprising at least one therapeutic agent and/or at least one diagnostic agent within the solid oral dosage form.

11. The solid oral dosage form according to one or more of claims 1 to 10 for use in medicine.

12. A solid oral dosage form according to any one of claims 1 to 11 for use in the oral delivery of at least one therapeutic agent and/or at least one diagnostic agent.

**Patentansprüche**

1.  Feste, orale Dosierungsform umfassend Liposomen, wobei die Liposomen ein Konjugat von

    (a) mindestens einem Typ von zellpenetrierendem Peptid (CPP), und
    (b) einer Verbindung, ausgewählt aus einem Lipid und einer Fettsäure, umfassen;

    wobei das Konjugat Teil der Liposomen-Lipiddoppelschicht ist;
    wobei die Dosierungsform eine magenresistente feste, orale Dosierungsform ist.

2.  Feste, orale Dosierungsform nach Anspruch 1, wobei die Liposomen in einer magensaftresistent überzogenen Kapsel oder magensaftresistent überzogenen Tablette enthalten sind.

3.  Feste, orale Dosierungsform gemäß Anspruch 1 oder 2, ferner umfassend mindestens einen pharmazeutisch verträglichen Exzipienten, und/oder mindestens einen Proteaseinhibitor, und/oder mindestens einen Lipaseinhibitor in der festen, oralen Dosierungsform.

4.  Feste, orale Dosierungsform gemäß einem oder mehr der Ansprüche 1 bis 3, wobei der mindestens eine Typ von CPP ein cyclisiertes CPP ist.

5.  Feste, orale Dosierungsform gemäß einem oder mehr der Ansprüche 1 bis 4, wobei der mindestens eine Typ von CPP aus der Gruppe ausgewählt ist, welche aus linearem oder cyclisiertem Penetratin, linearem oder cyclisiertem Transaktivator von Transkription (TAT)-Peptid, linearem oder cyclisiertem amphiphatischem Modellpeptid (MAP), linearem oder cyclisiertem R9, linearem oder cyclisiertem pVEC, linearem oder cyclisiertem Transportan, linearem oder cyclisiertem MPG, Kombinationen davon, und Dimeren davon besteht.

6. Feste, orale Dosierungsform gemäß einem oder mehr der Ansprüche 1 bis 5, wobei die Liposomen den mindestens einen Typ von CPP in einer Menge von 0,05 bis 5 Mol-%, bezogen auf die Lipid und/oder Fettsäure-Gesamtmenge, umfassen.

7. Feste, orale Dosierungsform gemäß einem oder mehr der Ansprüche 1 bis 6, wobei die Verbindung, an welche der mindestens eine Typ von CPP konjugiert ist, aus der Gruppe ausgewählt ist, welche aus Cholesterin und Derivaten davon, Phospholipiden, Lysophospholipiden, und Kombinationen davon besteht, wobei die Cholesterinderivate Steroide und Verbindungen mit einer Steroidmolekülgrundstruktur sind.

8. Feste, orale Dosierungsform gemäß einem oder mehr der Ansprüche 1 bis 7, wobei der mindestens eine Typ von CPP an die Verbindung über einen bifunktionellen PEG-Linker konjugiert ist.

9. Feste, orale Dosierungsform gemäß einem oder mehr der Ansprüche 1 bis 8, wobei die Liposomen lyophilisiert sind.

10. Feste, orale Dosierungsform gemäß einem oder mehr der Ansprüche 1 bis 9, ferner umfassend mindestens ein therapeutisches Mittel und/oder mindestens ein diagnostisches Mittel in der festen, oralen Dosierungsform.

11. Feste, orale Dosierungsform gemäß einem oder mehr der Ansprüche 1 bis 10 zur Verwendung in Medizin.

12. Feste, orale Dosierungsform gemäß irgendeinem der Ansprüche 1 bis 11 zur Verwendung in der oralen Applikation von mindestens einem therapeutischen Mittel und/oder mindestens einem diagnostischen Mittel.

**Revendications**

1. Forme posologique orale solide comprenant des liposomes, lesdits liposomes comprenant un conjugué de

   (a) au moins un type de peptides de pénétration cellulaire (CPP) et
   (b) un composé, choisi parmi un lipide et un acide gras ;

   dans laquelle ledit conjugué fait partie de la bicouche lipidique du liposome ;
   dans laquelle la forme posologique est une forme posologique orale solide gastro-résistante.

2. Forme posologique orale solide selon la revendication 1, dans laquelle lesdits liposomes sont compris dans une gélule à enrobage entérique ou un comprimé à enrobage entérique.

3. Forme posologique orale solide selon la revendication 1 ou 2, comprenant en outre au moins un excipient pharmaceutiquement acceptable et/ou au moins un inhibiteur de protéase et/ou au moins un inhibiteur de lipase dans la forme posologique orale solide.

4. Forme posologique orale solide selon une ou plusieurs des revendications 1 à 3, dans laquelle ledit au moins un type de CPP est un CPP cyclisé.

5. Forme posologique orale solide selon une ou plusieurs des revendications 1 à 4, dans laquelle ledit au moins un type de CPP est choisi dans le groupe consistant en une pénétratine linéaire ou cyclisée, un peptide de transactivation de transcription (TAT) linéaire ou cyclisée, un peptide modèle amphiphile (MAP) linéaire ou cyclisé, le R9 linéaire ou cyclique, le pVEC linéaire ou cyclisé, le transportane linéaire ou cyclisé, le MPG linéaire ou cyclisé, leurs combinaisons et leurs dimères.

6. Forme posologique orale solide selon une ou plusieurs des revendications 1 à 5 dans laquelle lesdits liposomes comprennent ledit au moins un type de CPP en une quantité comprise entre 0,05 et 5 mol% par rapport à la quantité totale de lipides et/ou d'acides gras.

7. Forme posologique orale solide selon une ou plusieurs des revendications 1 à 6, dans laquelle le composé avec lequel ledit au moins un type de CPP est conjugué est choisi dans le groupe consistant en le cholestérol et ses dérivés, les phospholipides, les lysophospholipides et leurs combinaisons, dans lequel les dérivés de cholestérol sont les stéroïdes et les composés présentant une structure moléculaire stéroïde de base.

8. Forme posologique orale solide selon une ou plusieurs des revendications 1 à 7, dans laquelle ledit au moins un type de CPP est conjugué audit composé par l'intermédiaire d'un agent de liaison PEG bifonctionnel.

9. Forme posologique orale solide selon une ou plusieurs des revendications 1 à 8, dans laquelle lesdits liposomes sont lyophilisés.

10. Forme posologique orale solide selon une ou plusieurs des revendications 1 à 9, comprenant en outre au moins un agent thérapeutique et/ou au moins un agent de diagnostic au sein de la forme posologique orale solide.

11. Forme posologique orale solide selon une ou plusieurs des revendications 1 à 10, destinée à être utilisée en médecine.

12. Forme posologique orale solide selon une ou plusieurs des revendications 1 à 11, destinée à être utilisée dans l'administration orale d'au moins un agent thérapeutique et/ou d'au moins un agent de diagnostic.

Figures

Figure 1

Figure 2

Figure 3

Figure 4

Figure 5

Figure 6

**Zetapotential with different concentrations of Penetratin**

Figure 7

**Zetapotential with different concentrations of MAP**

Figure 8

Figure 9

Figure 10

**Figure 11**

Structure conjugate:

Name lipid used for coupling:
(2R)-3-((((4,46-dioxo-46-(2,3,5,6-tetrafluorophenoxy)-
7,10,13,16,19,22,25,28,31,34,37,40,43-tridecaoxa-3-
azahexatetracontyl)oxy)(hydroxy)phosphoryl)oxy)propane-1,2-diyl distearate
➤ C77H138F4NO24P

**Figure 12**

**Name of modified lipid:**
Mal-dPEG™(12)-DSPE
**Chemical name:** (2R)-3-((((46-(2,5-dioxo-2,5-dihydro-1H-pyrrol-1-yl)-4,44-dioxo-7,10,13,16,19,22,25,28,31,34,37,40-dodecaoxa-3,43-diazahexatetracontyl)oxy)(hydroxy)phosphoryl)oxy)propane-1,2-diyl distearate

**Structure of modified lipid:**

Figure 13

Figure 14

Figure 15

Figure 16

**Figure 17**

## Zetapotential of liposomal formulations

**Figure 18**

Figure 19

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SILVIA F et al.** Matrix liposomes: A solid liposomal formulation for oral administration : Solid liposomes for oral administration. *Eur. J. Lipid Sci. Technol.,* 2014, vol. 116, 1145-1154